Europäisches Patentamt

European Patent Office

Office européen des brevets

(11) Publication number: **0 418 646 A2**

## EUROPEAN PATENT APPLICATION

(21) Application number: 90117034.0

(51) Int. Cl.⁵: **C07C 231/06, B01J 23/84**

(22) Date of filing: 05.09.90

(30) Priority: 18.09.89 HU 485489

(43) Date of publication of application:
27.03.91 Bulletin 91/13

(84) Designated Contracting States:
AT BE CH DE DK ES FR GB GR IT LI LU NL SE

(71) Applicant: MTA Központi Kémiai Kutato
Intézete
Pusztaszeri u. 59/67
H-1025 Budapest(HU)

Applicant: NITROKEMIA IPARTELEPEK
Pf. 45
H-8184 Füzfögyártelep(HU)

(72) Inventor: Paal, Julia Dr.
Pasaréti ut 99/a
H-1026 Budapest(HU)
Inventor: Putirszkaja, Galina Dr.
Bartha u. 4/b,
H-1126 Budapest(HU)
Inventor: Botar, Laszlo
Hegyalja u. 90/b,
H-1112 Budapest,(HU)
Inventor: Keszler, Agnes Dr.
Kövér Lajos tér 14,
H-1149 Budapest(HU)
Inventor: Nemes, Istvan Dr.
Tömös u. 20,
H-1112 Budapest,(HU)
Inventor: Vidoczy, Tamas Dr.

Bartok Béla u. 31,
H-1114 Budapest,(HU)
Inventor: Gal, Dezsö Dr.
Bérc u. 19/21,
H-1016 Budapest(HU)
Inventor: Vasvari, Gabor Dr.
Szamado u. 17,
H-1016 Budapest(HU)
Inventor: Metzger, Klara
Tancsics u. 1,
H-2084 Pilisszentivan(HU)
Inventor: Horvath, Maria
Vaskapu u. 35,
H-1097 Budapest,(HU)
Inventor: Sönfeld, Erzsébet
Matyashegyi u. 13,
H-1037 Budapest,(HU)
Inventor: Bandi, Agnes
Pollack M. u. 35,
H-2022 Tahi,(HU)
Inventor: Fodor, Jozsef
Haszkovo ut 17/c,
H-8220 Veszprém(HU)
Inventor: Rajnai, Kalman
Tancsics ut 81,
H-8220 Balatonalmadi,(HU)

(74) Representative: Beszédes, Stephan G., Dr.
Patentanwalt
Münchener Strasse 80a Postfach 1168
W-8060 Dachau(DE)

(54) **Process for the preparation of acyl amides by subjecting nitriles to catalytic hydration.**

(57) The invention relates to an improved process for the preparation of acyl amides by subjecting nitriles to hydration in the presence of a copper-containing catalyst. According to the invention hydration is performed in the presence of a catalyst prepared by admixing an aqueous solution comprising an alkali metal vanadate and an alkali metal silicate in a weight ratio of 1 : (1-2.5) with 0.1 to 1.2 moles, calculated for 1 mole of alkali metal silicate, of a water soluble magnesium and/or calcium compound and with a water soluble copper(II) compound to adjust the weight ratio of $V^V$ : $Cu^{II}$, expressed as metal, to (1:0.3) to (1:4.5), separating the resulting precipitate, admizing 0.7 to 3 parts by weight, calculated for 1 part by weight of $Cu^{II}$ ion, of metallic copper to the wet precipitate and drying the resulting mixture at a temperature not exceeding 200°C.

# PROCESS FOR THE PREPARATION OF ACYL AMIDES BY SUBJECTING NITRILES TO CATALYTIC HYDRATION

The invention relates to a process for the preparation of acyl amides by subjecting nitriles to catalytic hydration. The process of the invention can be applied particularly preferably for the preparation of acryl amide by subjecting acrylonitrile to catalytic hydration. The invention also relates to a process for the preparation of catalysts applicable for the above purpose.

It is well known that nitriles can be converted into acryl amides when reacting them with water in the presence of copper or copper salt catalysts. The known methods for the hydretion of nitriles differ from one another primarily in the nature of the catalyst applied

US patent No. 3,381,034 discloses the use of copper(I) and copper(II) salts as catalysts, which can be used optionally in admixture with elementary copper. A disadvantage of the method is that the end-product is obtained with a low yield.

According to German patent No. 2,001,903 reduced copper oxide reduced copper-chromium oxide reduced copper-molybdenum oxide or a mixture thereof is applied as catalyst. The use of these catalysts involves the disadvantage that the selectivity of the reaction is poor; thus e.g. when hydrating acrylonitrile or methacrylonitrile, ca. 30 % of the nitrile converts into $\beta$-hydroxy-propionitrile instead of the required acyl amide.

German patent No. 2,036,126 discloses the use of Raney copper, Ullmann's copper, metallic copper and various reduced copper compounds as catalysts. A common disadvantage of all of these catalysts is that they deactivate relatively quickly, and the catalysts should be reactivated in hydrogen atmosphere at elevated temperatures, which is a risky and uneconomical operation.

According to the method disclosed in German patent No, 2,320,060, the above disadvantages are overcome by utilizing a catalyst comprising copper and a magnesium silicate precipitated from an alkali silicate with a magnesium compound in the presence of a copper compound, said catalyst being pre-treated with a reducing gas, particularly hydrogen, at an elevated temperature, Copper can be admixed to the catalyst after the precipitation of magnesium silicate but prior to reduction, it is more pfererable, however, to form the entire copper content of the catalyst in the reduction step by reducing the copper compounds present when precipitating the magnesium silicate, It is mentioned in the specification that the catalyst may also contain, beside metallic copper, ionic copper (ie. copper /I/ and copper/II/ ions) in an amount of 0.1 to 30 % by weight, preferably 0.1 to 10 % by weight, calculated for the total copper content. The catalyst may also contain minor amounts of other metals, among others, vanadium, preferably in the form of metal compounds. The amount of such metal additives is preferably 1-30 % by weight calculated for the weight of magnesium present; the respective metal compounds are added usually to the reaction mixture applied in the precipitation of magnesium silicate.

Although the method disclosed in German patent No, 2,320,060 has several advantages (thus e.g. the catalyst is appropriately selective and has a relatively long life span), it is a disadvantage that the catalyst should he pre-treated with a reducing gas at a relatively high temperature. This operation increases the material, operation and energy demands of the process considerably, which involves the increase in the preparation costs of the acyl amide prepared. According to Example 6 of the cited patent, when acrylonitrile is hydrated in an aqueous solution, the solution should be rendered oxygen-free in order to avoid the deactivation of the catalyst, This operation further increases the production costs.

German patent No. 2,702,014 describes a process for the hydration of nitriles into acyl amides in which neither the reduction of the catalyst is required nor the reaction mixture should be rendered oxygen-free. According to this method hydration is performed with a catalyst comprising copper and vanadium; when preparing the catalyst, a copper(I) salt or a mixture comprising at least two of metallic copper, copper(I) salt and copper(II) salt is added to the vanadete. If desired, the catalyst can be treated with 0.001 to 1 molar equivalents of an oxidizing agent calculated for the copper content.

All of the examples of the cited patent disclose a laboratory scale process, wherein an aqueous solution comprising a few percent of nitrile is reacted for 0.5 to 1 hour generally at 80°C, and the resulting product is analysed. The conversion and the selectivity are generally very satisfactory. It has not been investigated, however, whether the catalyst can be used repeatedly or not. Our investigations have revealed that the catalyst is in fact inapplicable for repeated use; its activity drops by about its half already after one reaction step, and it becomes completely inactive after three applications. Therefore, due to its short life span, the catalyst cannot be applied for large-scale production, including primarily fixed-bed catalytic reactors of continuous operation.

Our aim was to elaborate a method for the preparetion of acyl amides by subjecting nitriles to catalytic hydration in which neither a prior reduction of the catalyst is required nor the reaction mixture should be rendered oxygen- free, and the process can be carried out for a prolonged period of time in a continuous operation without any appreciable decrease in the activity of the catalyst.

These aims are attained according to the invention by utilizing a specially prepared copper-containing catalyst in the hydration of nitriles.

The invention relates to an improved process for the preparation of acyl amides by subjecting nitriles to hydration in the presence of a copper-containing catalyst. According to the invention hydration is performed in the presence of a catalyst prepared by admixing an aqueous solution comprising an alkali metal vanadate and an alkali metal silicate in a weight ratio of 1 : (1-2.5) with 0.1 to 1.2 moles, calculated for 1 mole of alkali metal silicate, of a water soluble magnesium and/or calcium compound and with a water soluble copper(II) compound to adjust the weight ratio of $V^V$ : $Cu^{II}$; expressed as metal, to (1:0.3) to (1:4.5), separating the resulting precipitate, admixing 0.7 to 3 parts by weight, calculated for 1 part by weight of $cu^{II}$ ion, of metallic copper to the wet precipitate and drying the resulting mixture at a temperature not exceeding 200°C.

The invention also relates to a method for the preparation of a catalyst applicable in the hydration of nitriles into acyl amides. According to the invention the catalyst is prepared by admixing an aqueous solution comprising an alkali metal vanadate and an alkali metal silicate in a weight ratio of 1 : (1-2.5) with 0.1 to 1.2 moles, calculated for 1 mole of alkali metal silicate, of a water soluble magnesium and/or calcium compound and with a water soluble copper(II) compound to adjust the weight ratio of $V^V$ : $Cu^{II}$, expressed as metal, to (1:0.3) to (1:4.5) separating the resulting precipitate, admixing 0.7 to 3 parts by weight, calculated for 1 part by weight of $Cu^{II}$ ion, of metallic copper to the wet precipitate and drying the resulting mixture at a temperature not exceeding 200°C.

The invention also relates to a catalyst prepared by the above method.

According to the invention sodium and/or potassium silicate can be applied as alkali metal silicate. Of the water soluble magnesium and/or calcium compounds applicable in the method of the invention magnesium nitrate, calcium nitrate, magnesium chloride, calcium chloride, magnesium sulfate, magnesium acetate, magnesium iodide and magnesium bromide are to be mentioned. When both magnesium and calcium compounds are applied, they can be utilized in an arbitrary ratio. The magnesium and/or calcium compound contacted with the alkali metal silicate present forms a magnesium and/or calcium silicate, which serves as support for the catalyst. This support contains the $Cu^{II}$ and $V^V$ compounds in a very fine distribution. When admixing this $Cu^{II}$ and $V^V$ containing support with metallic copper, a catalyst with the following characteristic composition is obtained:

| | |
|---|---|
| $V^V$ | 7.5 to 13 % by weight |
| $Cu^{II}$ | 13 to 31 % by weight |
| support | 9 to 17 % by weight |
| metallic copper | 20 to 38 % by weight |

vanadate/support weight ratio: 2 to 4
$Cu^{II}$/support weight ratio: 1 to 2.5 metallic copper/support weight ratio: 1.5 to 3
$Cu^{II}$/metallic copper weight ratio: 0.7 to 3

It appears from the above that the catalyst prepared according to the invention differs not only in its way of preparation (omission of reduction) but also in its composition from that disclosed in German patent No, 2,320,060. The catalyst utilized in the method of the invention comprises much more vanadate than that disclosed in the cited reference; namely vanadate is only an optional component of the known catalyst, and if present, its amount is only a fraction of the amount of the magnesium content of the support. The catalyst utilized in the method of the invention comprises much more ionic copper than the known ones, Considering this fact, it is even more striking that in the method of the invention there is no need for the reduction of the catalyst, and the catalyst retains its activity for a prolonged period of time even without performing the reaction under oxygen-free atmosphere.

Apart from the nature of the catalyst, the hydration of nitriles according to the invention is performed in a manner known per se, e.g. as disclosed in the references cited above, Fixed-bed catalyst and suspended catalyst can equally be used, but from technological aspects it is more preferred to use a fixed catalyst bed. In this instance the outstandingly high life span of the catalyst is a particular advantage. The method of the invention can also be combined with the method disclosed in Hungarian patent No, 180,855, according to which hydration is performed in the presence of an emulsifying agent,

The main advantages of the method of the invention, compared to the known ones, are as follows:

- the life span of the catalyst is outstandingly high; no appreciable activity loss can be observed even after a reaction time of 4500 hours;
- the reduction of the catalyst is not necessary, therefore the preliminary reduction step involving serious technological difficulties and high production costs can be avoided;
- the reaction mixture need not be rendered oxygen-free, which involves a significant simplification of technology and a considerable reduction of operation expenses.

The invention is elucidated in detail by the aid of the following non-limiting Examples.

Example 1

4 g of NaOH were dissolved in 240 ml of water, and 3 g of $V_2O_5$ were added to the resulting solution under stirring. After the decolouration of the solution, a mixture of 15 g of sodium silicate (water glass) and 85 ml of water were added

7 g of $Mg(NO_3)_2.6H_2O$ were dissolved in 20 ml of water, and 10 g of $CuCl_2.2H_2O$ were dissolved in 40 ml of water. The two aqueous solutions were combined, and then added dropwise into the above solution. The precipitate formed was filtered off, and 5 g of metallic copper were mixed to the wet precipitate. The resulting mass was extruded and dried at 160°C. 17 g of catalyst were obtained.

The estimated composition of the catalyst was as follows:

| Na | 13.5 % by weight | |
|---|---|---|
| $V^{V}$ | 10.0 % by weight | |
| O | 11.8 % by weight | |
| $Cu^{II}$ | 22.4 % by weight | |
| | $\overline{57.7}$ % by weight | |
| support | 12.9 % by weight | (Mg: 4.1 % by wt) |
| metallic copper | 29.4 % by weight | |

$V^{V}$/support weight ratio: 2.73
$Cu^{II}$/support weight ratio: 1.73
metallic copper/support weight ratio: 2.27

Example 2

The process of Example 1 was followed with the difference that 3 g of $CaCl_2$ was utilized instead of 7 g of $Mg(NO_3)_2.6H_2O$.

The estimated composition of the catalyst was as follows:

| Na | 13.5 % by weight | |
|---|---|---|
| $V^{V}$ | 10.0 % by weight | |
| O | 11.8 % by weight | |
| $Cu^{II}$ | 22.4 % by weight | |
| support | 12.9 % by weight | (Ca: 6.45 % by wt) |
| metallic copper | 29.4 % by weight | |

Example 3

This example is given for comparison purposes.

The catalyst prepared as described in German patent No. 2,702,014 was tested in a series of reactions.

The catalyst was prepared by dissolving 9.6 g of NaOH in 600 ml of water, adding 7.28 g of $V_2O_5$, and after the dissolution of this component introducing 20.4 g of $CuCl_2.2H_2O$ as an aqueous solution 0.25 g of the resulting catalyst component was admixed with 0.25 g of copper powder, and the resulting mixture was applied as catalyst in the hydration of acrylonitrile

5 ml of a 6 % by weight aqueous acrylonitrile solution were poured onto the catalyst, the reaction mixture was stirred at 80°C for 1 hour, and the catalyst was filtered off. The filtrate contained 3 % by weight of acryl amides.

5 ml of a 6 % by weight aqueous acrylonitrile solution were poured onto the recovered catalyst, the reaction mixture was stirred again at 80°C for 1 hour, and the catalyst was filtered off. The filtrate contained 1.4 % by weight of acryl amide.

In the third reaction again 5 ml of a 6 % by weight aqueous acrylonitrile solution were poured onto the recovered catalyst, the reaction mixture was stirred at 80°C for 1 hour, and the catalyst was filtered off. No acryl amide could be detected in the filtrate.

Example 4

10 ml of a 6 % by weight aqueous acrylonitrile solution were added to 0.5 g of the catalyst prepared as described in Example 1. The reaction mixture was stirred at 80°C for 1 hour, and the catalyst was filtered off. The filtrate contained 2.5 % by weight of acryl amide.

10 ml of a 6 % by weight aqueous acrylonitrile solution were poured onto the recovered catalyst, and the reaction mixture was stirred again at 80°C for 1 hours. The catalyst was filtered off. The filtrate contained 2.0 % by weight of acryl amide.

The recovered catalyst was used in a further hydration reaction as described above. The filtrate contained 2.2 % by weight of acryl amide.

The recovered catalyst was used fourth time in the preparation of acryl amide as described above. The filtrate contained 0.8 % by weight of acryl amide.

The recovered catalyst was used again (fifth time) in the preparation of acryl amide as described above. The filtrate contained 0.8 % by weight of acryl amides.

Example 5

10 ml of a 6 % by weight aqueous acrylonitrile solution were added to 0.5 g of the catalyst prepared as described in Example 2, and the reaction mixture was stirred at 70°C for 2 hours. The catalyst was filtered off. The filtrate contained 2.7 % by weight of acryl amide.

Example 6

9.8 g of the catalyst prepared as described in Example 1 were filled into a flow-through reactor with a working capacity of 14 ml. A 6 % by weight aqueous solution of acrylonitrile was passed through the reactor by gravity. The temperature of the reactor was adjusted to 75-80°C. The effluent was analyzed to determine its acryl amide content. The results are listed in Table I.

TABLE I

| Time (hours) | g of acryl amide/kg of catalyst x hour | Time (hours) | g of acryl amide/kg of catalyst x hour |
|---|---|---|---|
| 21 | 23.7 | 634 | 18.3 |
| 122 | 26.9 | 724 | 23.8 |
| 195 | 17.6 | 825 | 17.9 |
| 309 | 18.6 | 885 | 15.3 |
| 391 | 17.4 | 978 | 12.6 |
| 490 | 21.6 | 1168 | 13.9 |
| 562 | 18.3 | 1313 | 14.7 |

Example 7

153 g of the catalyst prepared as described in Example 1 were filled into a flow-through reactor, and a 25 % by weight aqueous solution of acrylonitrile was passed through the reactors. Considering that only 7 % by weight of acrylonitrile is soluble in water, 1 g/l of dodecylsulfonic acid emulsifying agent was added to the mixture to obtain a homogeneous solution. The reaction temperature was adjusted to 80°C. The results are listed in Table II.

Table II

| Time (hours) | g of acryl amide/kg of catalyst x hour | Time (hours) | g of acryl amide/kg of catalyst x hour |
|---|---|---|---|
| 240 | 40.0 | 2400 | 36.6 |
| 480 | 48.1 | 2640 | 23.5 |
| 720 | 25.0 | 2880 | 16.7 |
| 960 | 29.8 | 3120 | 14.5 |
| 1200 | 25.9 | 3600 | 26.6 |
| 1440 | 36.7 | 3840 | 20.4 |
| 1680 | 16.0 | 4080 | 16.1 |
| 1920 | 30.5 | 4320 | 25.9 |
| 2160 | 29.0 | 4560 | 27.7 |

Example 8

The process described in Example 7 was followed with the deference that an aqueous solution comprising 7 % by weight of acetonitrile was passed through the reactor. The starting substance was converted into acetamide. The results are listed in Table III.

Table III

| Time (hour) | g of acetamide/g of catalyst x hour | Time (hour) | g of acetamide/g of catalyst x hour |
|---|---|---|---|
| 240 | 20.2 | 1200 | 15.4 |
| 480 | 14.2 | 1440 | 15.4 |
| 720 | 14.5 | 1680 | 13.8 |
| 960 | 14.7 | | |

Example 9

The process of Example 1 was followed with the difference that the following amounts were used:

| NaOH | 4 g |
|---|---|
| $V_2O_5$ | 3 g |
| Sodium silicate (water glass) | 16 g |
| $Mg(NO_3)_2.6H_2O$ | 7.5 g |
| $CuCl_2.2H_2O$ | 14.0 g |
| Metallic copper | 3.3 g |

The estimated composition of the catalyst was as follows:

| Na | 13.6 % by weight |
|---|---|
| $V^V$ | 9.9 % by weight |
| O | 12.6 % by weight |
| support | 13.8 % by weight |
| $Cu^{II}$ | 30.6 % by weight |
| metallic copper | 19.5 % by weight |

vanadate/support weight ratio: 2.5
$Cu^{II}$/support weight ratio: 2.2
metallic copper/support weight ratio: 1.4

## Example 10

The process of Example 1 was followed with the difference that the following amounts were used:

| NaOH | 3 g |
|---|---|
| $V_2O_5$ | 2.25 g |
| Sodium silicate (water glass) | 15 g |
| $Mg(NO_3)_2.6H_2O$ | 6.0 g |
| $CuCl_2.2H_2O$ | 10.0 g |
| Metallic copper | 6.3 g |

The estimated composition of the catalyst was as follows:

| Na | 10.2 % by weight |
|---|---|
| $V^V$ | 7.4 % by weight |
| O | 9.5 % by weight |
| $Cu^{II}$ | 21.8 % by weight |
| support | 13.3 % by weight |
| metallic copper | 37.0 % by weight |

vanadate/support weight ratio: 2.0
$Cu^{II}$/support weight ratio: 1.6
metallic copper/support weight ratio: 2.8

## Example 11

The process of Example 1 was followed with the difference that the following amounts were used:

| NaOH | 3 g |
|---|---|
| $V_2O_5$ | 2.25 g |
| Sodium silicate (water glass) | 15 g |
| $Mg(NO_3)_2.6H_2O$ | 5.0 g |
| $CuCl_2.2H_2O$ | 14.0 g |
| Metallic copper | 5.0 g |

The estimated composition of the catalyst was as follows:

| Na | 10.2 % by weight |
|---|---|
| $V^V$ | 7.4 % by weight |
| O | 9.5 % by weight |
| $Cu^{II}$ | 30.6 % by weight |
| support | 12.8 % by weight |
| metallic copper | 29.4 % by weight |

vanadate/support weight ratio: 2.1 $Cu^{II}$/support weight ratio: 2.4 metallic copper/support weight ratio: 2.3

Example 12

The process of Example 1 was followed with the difference that the following amounts were used:

| NaOH | 5.2 g |
|---|---|
| $V_2O_5$ | 3.9 g |
| Sodium silicate (water glass) | 15 g |
| $Mg(NO_3)_2.6H_2O$ | 6.0 g |
| $CuCl_2.2H_2O$ | 10.0 g |
| Metallic copper | 3.3 g |

The estimated composition of the catalyst was as follows:

| Na | 17.6 % by weight |
|---|---|
| $V^V$ | 12.8 % by weight |
| O | 16.5 % by weight |
| $Cu^{II}$ | 21.8 % by weight |
| support | 11.9 % by weight |
| metallic copper | 19.5 % by weight |

vanadate/support weight ratio: 3.95
$Cu^{II}$/support weight ratio: 1.8
metallic copper/support weight ratio: 1.6

**Claims**

1. process for the preparation of acyl amides by subjecting nitriles to hydration in the presence of a copper-containing catalyst, characterized in that hydration is performed in the presence of a catalyst prepared by admixing an aqueous solution comprising an alkali metal vanadate and an alkali metal silicate in a weight ratio of 1 : (1-2.5) with 0.1 to 1.2 moles, calculated for 1 mole of alkali metal silicate, of a water soluble magnesium and/or calcium compound and with a water soluble copper(II) compound to adjust the weight ratio of $V^V$ : $Cu^{II}$, expressed as metal, to (1:0.3) to (1:4.5), separating the resulting precipitate, admixing 0.7 to 3 parts by weight, calculated for 1 part by weight of $Cu^{II}$ ion, of metallic copper to the wet precipitate and drying the resulting mixture at a temperature not exceeding 200°C.

2. A process as claimed in claim 1, characterized in that a catalyst comprising $V^V$ and $Cu^{II}$ ions in a weight ratio of 1 : (0.3-2) is applied.

3. A process for the preparation of a catalyst applicable in the hydràtion of nitriles into acyl amides, characterized by admixing an aqueous solution comprising an alkali metal vanadate and an alkali metal silicate in a weight ratio of 1 : (1-2.5) with 0.1 to 1.2 moles, calculated for 1 mole of alkali metal silicate, of a water soluble magnesium and/or calcium compound and with a water soluble copper(II) compound to adjust the weight ratio of $V^V$ : $Cu^{II}$, expressed as metal, to (1:0.3) to (1:4.5), separating the resulting precipitate, admixing 0.7 to 3 parts by weight, calculated for 1 part by weight of $Cu^{II}$ ion, of metallic copper to the wet precipitate and drying the resulting mixture at a temperature not exceeding 200°C.

4. catalyst whenever prepared as disclosed in claim 3.